# EUROPÄISCHE PATENTANMELDUNG

(11) **EP 0 666 064 A1**
(43) Veröffentlichungstag der Anmeldung: **09.08.1995**
(21) Anmeldenummer: 94118749.4
(22) Anmeldetag: 29.11.1994
(51) Int. Cl.: A61F 2/00

(54) **Schutzvorrichtung für den unkontrollierten Abfluss von Urin**

(30) Priorität: 08.02.1994 DE 9402017 U
(71) Anmelder: Beyschlag, Heinz Georg, D-73529 Schwäbisch Gmünd (DE)
(72) Erfinder:

(57) **Zusammenfassung**

Eine Schutzvorrichtung für den unkontrollierten Abfluß von Urin bei Inkontinenz. Ein, in einer Harnröhre (1) anordenbarer länglicher Verschluß (2,5) ist von einem Hohlkörper (2) gebildet. Der Hohlkörper (2) ist an seinem proximalen Ende (4) offen und am seinem distalen Ende (3) geschlossen ausgebildet.

## Beschreibung

Die Erfindung betrifft eine Schutzvorrichtung für den unkontrollierten Abfluß von Urin bei Inkontinenz mit einem, in einer Harnröhre anordenbaren länglichen Verschluß, der von einem Hohlkörper gebildet ist, der an einem Ende offen ausgebildet ist.

Inkontinenz, also das Unvermögen Urin bzw. Harn willentlich zurückzuhalten und zu entleeren, ist eine Krankheit, die betroffene Menschen in ihrem Lebensrhythmus stark beeinträchtigt. Diese Menschen sind in erheblichem Maße auch psychischen Belastungen ausgesetzt. Es gibt zwar eine ganze Reihe von technischen Hilfsmitteln für diese Patienten, aber durch die Individualität jedes einzelnen gibt es immer neue Aufgaben, die gelöst werden müssen. Darüberhinaus muß auch der derzeitige Stand der Technik weiterentwickelt, also verbessert werden.

Für die Inkontinenzversorgung stehen heute drei verschiedene Arten von Hilfsmitteln zur Verfügung:
1. die absorbierenden Hilfsmittel,
2. die externen Auffangsysteme für den Urin,
3. die im Körper angeordneten Urinableitungssysteme.

Aus der DE-OS 19 57 693 ist ein Verschluß für eine Harnröhre bekannt. Er ist von einem kurzen, schlanken, dehnbaren, am proximalen Ende geschlossenen Schlauch aus Weichgummi gebildet, der in die männliche Harnröhre eingeführt wird. Im Inneren des Schlauches ist eine Halteeinrichtung angeordnet, die vom distalen, offenen Ende in den Schlauch hinein angeordnet ist und die über ein, über das Schlauchende überstehendes Kopfteil bedient wird. Die Halteeinrichtung mit dem Kopfteil ist fest mit dem Schlauch verbunden. Durch Drehung einer, im Kopfteil angeordneten Schraube, wird mit Hilfe einer Anzahl von Spreizhebeln der Schlauch so weit gedehnt, daß der Verschluß in der Harnröhre festgehalten wird und "ein völlig ausreichender Verschluß der Harnröhre bewirkt wird" (Seite 3, Zeile 17,18 der Beschreibung).

Dieser Stand der Technik hat den Nachteil, daß er nicht dicht ist. Die Spreizhebel bilden bei der Dehnung des Schlauches ein Polygon. Zwischen den Anlegepunkten werden zwei parallele Flächen gebildet, die nicht dicht sein können. Zum Dichten müßten die Spannkräfte unendlich groß sein. Weiterhin ist von Nachteil, daß der Kopf 3 mit der Schraube 4 über das Ende der Harnröhre herausragt und beim Tragen stört. Einmal ist es das Gewicht, das auf die Harnröhre und deren Umgebung einwirkt und schmerzt. Andererseits werden Schmerzen durch Reibung z.B.am Glied und der Eichel beim Mann hervorgerufen.

Es ist die Aufgabe der vorliegenden Erfindung eine gattungsgemäße Schutzvorrichtung so zu verbessern, daß diese benutzerfreundlich ohne fremde Hilfe vom Anwender selbst leicht und problemlos anwendbar ist, die leicht von Gewicht ist und bei optimalem Tragekomfort einen sicheren und absolut flüssigkeitsdichten Sitz in der Harnröhre aufweist und die umweltfreundlich und wirtschaftlich ist.

Diese Aufgabe wird erfindungsgemäß dadurch gelöst, daß der Hohlkörper an seinem distalen Ende geschlossen und an seinem proximalen Ende offen ausgebildet ist.

Der so ausgebildete Verschluß ist so in der Harnröhre angeordnet, daß das offene Ende des Hohlkörpers in Richtung Blase geöffnet und das geschlossene Ende des Hohlkörpers am distalen Ende der Harnröhre bei der Eichel angeordnet ist. Der Verschluß hat keine eingebauten Teile und ist deshalb von niedrigen Gewicht und ist leicht vom Benutzer zu handhaben.

In Ausgestaltung der Erfindung kann der Hohlkörper annähernd die Form eines elliptischen Paraboloids aufweisen; er kann aber ebensogut annähernd zylinder- oder kegelförmig ausgebildet sein. Der Hohlkörper kann dabei eine Länge aufweisen, die nicht länger als die Harnröhre ist. Er ist so in der Harnröhre angeordnet, daß sein geschlossenes, distales Ende flächig an dem Halsteil der Harnröhre anliegt. In vorteilhafter Weiterbildung kann der Hohlkörper aus einem elastischen Material gebildet sein und eine Wandstärke aufweisen, die im Bereich des geschlossenen Endes dicker, als im Bereich des offenen Endes ausgebildet ist. Schließlich kann der Hohlkörper am geschlossenen Ende eine Zugeinrichtung, insbesondere einen Faden aufweisen.

Es hat sich überraschenderweise gezeigt, daß der Erfindungsgegenstand mit einem, am proximalen Ende, also "nach hinten offenen" länglichen Hohlkörper, aus elastischem Material, einen optimal festen Sitz in der Harnröhre hat und absolut dicht an der Harnröhrenwand anliegt. Vermutlich ist das darauf zurückzuführen, daß der Druck in der Blase sich über die Flüssigkeitssäule in der Harnröhre bis in den Innenraum des Hohlkörpers überträgt und die Wand des Hohlkörpers aus elastischem Material an die Harnröhrenwand anpreßt. Die Anpreßkraft verändert sich proportional zum Flüssigkeitsdruck. Das bedeutet, daß sich mit steigendem Druck im Inneren des Hohlkörpers die Anpreßkraft für die Hohlkörperwand an die Harnröhrenwand im gleichen Verhältnis erhöht. Das verwendete Material ist hautverträglich und antiallergisch. Es ist recycelbar und deshalb umweltfreundlich.

Die längliche Form des Hohlkörpers ist annähernd als elliptischer Paraboloid, zylindrisch oder kegelförmig ausgebildet. Diese Ausgestaltung ist besonders vorteilhaft. Der Hohlkörper kann sich mit seiner ganzen elastischen Mantelfläche, an die relativ unebene Wand der Harnröhre anlegen. Die elastische Mantelfläche des Hohlkörpers, kann mit einer Vorspannung ausgestattet sein und wird dadurch, schon von Anfang an, an die Harnröhrenwand angepreßt und dichtend daran gehalten, auch bei anfänglich noch nicht vorhandenem Druck durch eine Flüssigkeitssäule. Dabei ist die Flexibilität des elastischen Materials von besonderem Vorteil, hinsichtlich der unregelmäßigen Oberfläche der Wand der Harnröhre. Der Hohlkörper sitzt deshalb von Anfang an fest und dicht. Beim Anwender treten beim Tragen des Verschlusses keine Schmerzen im Bereich der Harnröhre auf, wie es von anderen instrumentellen Einrichtungen her bekannt ist. Es wird vermutet, daß dies auf das Zusammenwirken von Flüssigkeitsdruck und Anpreßdruck zurückzuführen ist.

Der Hohlkörper kann jede beliebige Länge aufweisen, die maximal der der Harnröhre entspricht. In der Praxis wird er vorzugsweise von kurzer Länge sein. Es reicht völlig aus, den Hohlkörper zum sicheren Abdichten der Harnröhre nur wenige Zentimeter lang, beispielsweise 2 bis 5 cm auszuführen. Für Sonderfälle kann er länger sein. Der Durchmesser eines solchen Hohlkörpers ist vorgegeben und hängt vom Durchmesser der jeweiligen Harnröhre ab. Deshalb werden die Hohlkörper in verschiedenen Grössen hergestellt.

Wesentliche für die Erfindung ist, daß der Hohlkörper so gebaut ist, daß das distale Ende flächig an der Innenseite des Halsteils anliegt und nicht durch das Halsteil hindurch, an der Eichel, übersteht. Dies muß der Patient schon beim Einführen beachten. Konstruktionsseitig ist dieser Forderung dadurch Rechnung getragen, daß das geschlossene, distale Ende des Hohlkörpers dickwandig ist. Dadurch ist dieser Teil weniger zusammendrückbar und mit geringerer Elastizität ausgebildet und kann sich nicht oder nicht so leicht und unspürbar herausschieben. Für ein solches selbständiges Herausschieben des Hohlkörpers können Kräfte in Frage kommen, die von aussen auf die Harnröhre einwirken, wie z.B. Druck durch Sitzen.

Die vorgeschlagene unterschiedliche Wandstärke des elastischen Hohlkörpers ist deshalb wichtig, weil am distalen Ende der Hohlkörper eine geringere Zusammendrückbarkeit aufweisen muß, und weil beim Entfernen des Verschlusses mit dem Faden, ein sicheres Herhausziehen gewährleistet sein muß. Aus diesem Grund ist der Hohlkörper distalseitig dicker in der Wandstärke ausgeführt. Im Bereich des offenen, proximalen Endes besitzt der Hohlkörper eine dünnere Wandstärke, denn hier ist eine grosse Elastizität und Flexibilität des Materials Voraussetzung für guten Halt und sichere Abdichtung.

Die Erfindung ist von relativ einfacher Gestaltung, leicht herzustellen und löst die Aufgabe restlos. Weitere Vorteile, die die Erfindung mit sich bringt und auf die extra und besonders hinzuweisen wichtig erscheint, sind:
a) eine Infektionsgefahr, die bei intern im Körper angeordneten, bekannten Vorrichtungen, insbesondere bei Kathedern, mancherlei Probleme bereitet, ist mit der erfindungsgemäßen Vorrichtung - bei richtiger Anwendung - fast nicht mehr möglich.Der vorgeschlagene Verschluß wird in sterilem Zustand in die Harnröhre eingeführt. Zu jeder Blasenentleerung muß er entfernt, d.h. herausgezogen werden. Anschliessend wird ein neuer, steriler Verschluß eingesetzt. Bei durchschnittlich etwa vier- bis sechsmaliger Blasenentleerung pro Tag, ist somit ein Verschluß höchstens fünf Stunden im Einsatz. Eventuell in der Harnröhre eingenistete Krankheitskeime oder sonstige Infektionserreger werden bei jeder Blasenentleerung mit dem Urin aus der Harnröhre herausgespült. Das gefürchtete "Hochwachsen" von Keimen in die Blase und/oder in andere Organe, in diesem Bereich, ist mit der Erfindung nicht zu erwarten. Eine Blasenentzündung beispielsweise, verursacht durch die erfindungsgemäße Vorrichtung ist kaum denkbar. Auch eine Blasenerkrankung durch im Ableitungssystem "hochwachsende" Erreger ist gänzlich ausgeschlossen, weil es weder einen Beinbeutel, noch einen Katheder gibt.
b) Ein "Undichtwerden" des Verschlusses, möglicherweise durch Materialermüdung ist unmöglich, weil der Verschluß etwa nach längstens vier bis fünf Stunden Einsatz ausgetauscht wird.
c) Der wohl wichtigste Punkt in dieser Reihe und somit der größte Vorteil dieser Erfindung ist die Schaffung des "mobilen Patienten". Ein an Harninkontinenz leidender Mensch, mit dem vorgeschlagenen Verschluß, kann sich über längere Zeit völlig frei bewegen, ohne Behinderung durch irgendwelche weiteren Hilfsmittel, wie z.B. Windeleinlagen, Katheder mit Beinbeutel etc.. Unangenehme Geruchsentwicklung, wie insbesondere bei Verwendung von absorbierenden Systemen (Windelhosen), können mit der Erfindung nicht mehr auftreten. Mit der Erfindung ist der Kranke im Genitalbereich immer trocken. Es tritt keine Hautreizung mit Wundsein in diesem Bereich mehr auf. Der Patient kann sich wieder - ohne psychische Belastungen - selbstsicher unter anderen Menschen bewegen. Er kann wieder ungehindert am öffentlichen Leben teilnehmen; er braucht kein "Einsiedlerleben" mehr führen.
   "Mobiler Patient" bedeutet aber auch, daß viele bettlägerige Menschen nicht mehr ständig an das Bett gefesselt sind, nur weil ihnen ein Katheder angelegt ist. Häufig kann beim Einsatz eines vorgeschlagenen Verschlussen und bei regelmäßiger Blasenentleerung auf das Kathedersystem verzichtet werden.
   Bettlägerigkeit kann insbesondere bei älteren Menschen negative Begleiterscheinungen nach sich ziehen, die im schlimmsten Fall - hauptsächlich bei schwachen Personen - zum Tode führen kann, wie z.B. bei Lungenentzündung. Aber auch Beeinträchtigungen, insbesondere des Gehapparates, Appetitlosigkeit, Kreislaufstörung, Aufliegen etc. sind möglich. Ihre Behandlung kann eine nicht zu unterschätzende Kostenlawine auslösen. Bei einem "mobilen Patienten" sind solcherlei Gefahren jedoch gering.
   Ein ganz gravierender Vorteil ist, daß mache Patienten, die heute noch stationär im Krankenhaus einliegen müssen und sei es nur zur Beobachtung, daß diese Menschen Dank ihrer Mobilität das Krankenhaus bzw. den Arzt ambulant aufsuchen können. Insbesondere dieser letztgenannte Punkt dürfte eine, heute noch nicht vorhersehbare, Kosteneinsparung zur Folge haben.
   Die Liste von Vorteilen, hinsichtlich des "mobilen Patienten" ließe sich noch weiter fortsetzen. Wichtig ist einzig, daß mit dieser Erfindung Menschen geholfen wird, daß diese Menschen ihre schon verlorengegangen Selbstsicherheit wieder finden und daß sie sich wieder als vollwertige Menschen fühlen dürfen.
d) Hinsichtlich der Umweltfreundlichkeit sei soviel gesagt, daß der Erfindungsgegenstand selbst vom Volumen her klein und von geringem Material ist. Das Material kann aufbereitet und erneut verarbeitet werden. Eine Verpackung ist notwendig wegen der sterilen Aufbewahrung der Vorrichtung. Sie ist aber klein und problemlos zu entsorgen. Abfallberge, wie bei Einsatz von absorbierenden Hilfsmitteln (z.B.Windeln) , gibt es mit dem erfindungsgemäßen Verschluß nicht.
e) Die Wirtschaftlichkeit dieser Erfindung ergibt sich in einigen Punkten schon aus den vorstehenden Ausführungen. Deshalb soll an dieser Stelle nur auf die einfache Herstellbarkeit durch die Einstückigkeit des kompletten Verschlusses hingewiesen werden. Durch eine Produktion in Großserien ist der Erfindungsgegenstand kostengünstig. Die Verwendung eines solchen preiswerten Verschlusses kann manch anderes kostenintensives Hilfsmittel ersetzen.

Nachfolgend wird die Erfindung, anhand einer prinzipmässigen Zeichnung, an einem Ausführungsbeispiel erläutert.

Es zeigt
- Figur 1: den erfindungsgemäßen Verschluß in der Harnröhre angeordnet, in Seitenansicht stark vergrössert;
- Figur 2: eine perspektivische Ansicht des Verschlussen, ebenfalls vergrössert.

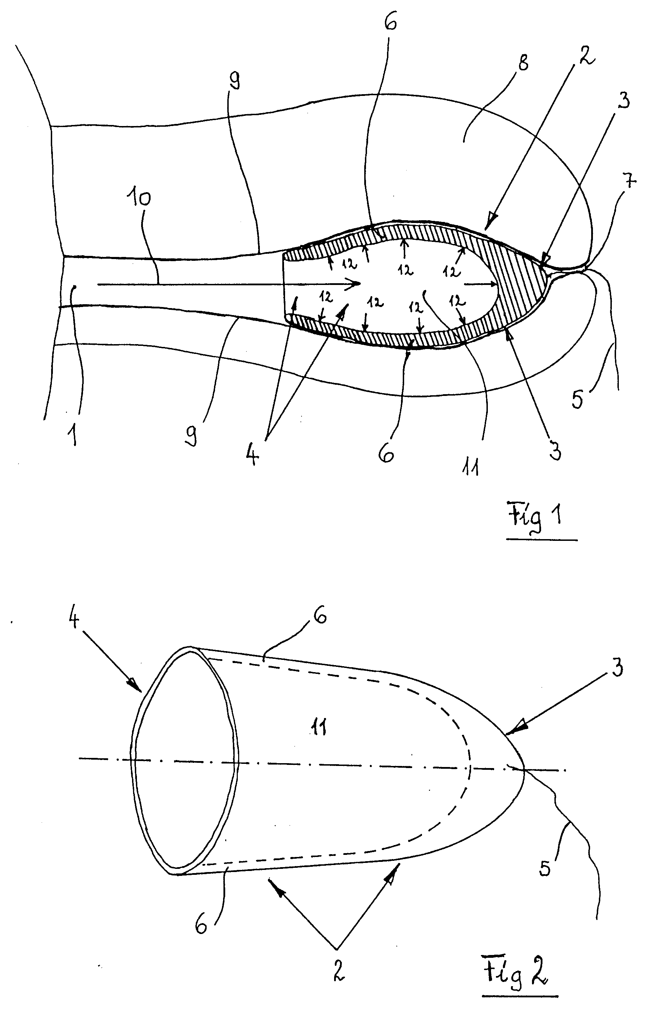

Eine Schutzvorrichtung für den unkontrollierten Abfluß von Urin bei Inkontinenz ist von einem länglichen Verschluß 2,5 gebildet, der in einer Harnröhre 1 angeordnet ist. Der Verschluß 2,5 ist von einem Hohlkörper 2 gebildet, an dem ein Faden 5 befestigt ist. Der Hohlkörper 2 ist an seinem distalen Ende 3 geschlossen und an seinem proximalen Ende 4 offen ausgebildet. Der Hohlkörper 2 ist kürzer, als die Länge der Harnröhre 1. Der Hohlkörper 2 ist in der Harnröhre 1 so angeordnet, daß er mit seinem distalen Ende 3 flächig am Halsteil 7 der Harnröhre 1 anliegt und sich in die Harnröhre 1 hinein erstreckt. Der Hohlkörper 2 hat die Form eines elliptischen Paraboloids. Die Form kann aber ebenso zylindrisch, kegel- oder kegelstumpfförmig sein. Der Hohlkörper 2 ist einstückig aus einem elastischen Material hergestellt.

Die Wandstärke des Hohlkörper 2 ist im Bereich des geschlossenen, distalen Endes 3 dicker, als im Bereich des offenen, proximalen Endes 4.

Beim Einführen des Verschlusses 2,5 in die Harnröhre 1 wird der Hohlkörper 2 am proximalen Ende 4 radial zusammmengedrückt z.B. mit den Fingern des Patienten. Das so geformte, schlauchähnliche, schlanke Gebilde wird in das begrenzt dehnbare Halsteil 7 der Eichel 8 eingeführt und der überstehende Teil des Hohlkörpers 2 nachgeschoben bis das distale Ende 3 des Hohlkörpers 2 ganz in der Harnröhre 1 steckt. Nur der Faden 5 hängt am Halsteil 7 heraus.

Die Wand 6 des Hohlkörpers 2 besitzt eine Vorspannung. Dadurch legt sich die Wand 6 von selbst an die Harnröhrenwand 9 an und dichtet in diesem Bereich die Harnröhre 1 ab. Ein Gesamtdruck 10, gebildet aus der Summe der Drücke aus Blase und Flüssigkeitssäule (symbolisiert durch den langen Pfeil 10) verteilt sich im Hohlraum 11 des Hohlkörpers 2 und übt einen Preßdruck 12 (symbolisiert durch die kurzen Pfeile 12) auf die Innenseite der Wand 6 und preßt diese Wand 6 gegen die Harnröhrenwand 9.

Bei leerer Blase und nicht vorhandener Flüssigkeitssäule reicht die Vorspannung in der Wand 6 aus, um den Hohlkörper 2 fest und dicht in der Harnröhre 1 zu halten. Mit zunehmendem Aufbau einer Flüssigkeitssäule in der Harnröhre 1 wird ein Gesamtdruck 10 aufgebaut, der als Preßdruck 12 die Wand 6 gegen die Harnröhrenwand 9 preßt.

Der Preßdruck 12 wird proportional zum Gesamtdruck 10 aufgebaut, d.h. je höher der Gesamtdruck 10 ist, desto höher ist der Preßdruck 12 und entsprechend stärker wird die Wand 6 an die Harnröhrenwand 9 gepreßt. So wird der Hohlkörper 2 immer dicht in der Harnröhre 1 festgehalten. Der Gesamtdruck 10, der senkrecht auf das distale Ende 3 im Hohlkörper 2 wirkt, ist zu gering, als daß in diesem Bereich das dickerwandige Material so verformt werden könnte, daß unter teilweiser Aufhebung des Preßdrucks 12 der Hohlkörper 2 durch das Halsteil 7 hindurchgepreßt werden könnte.

Zum Entfernen des Verschlusses 2,5 dient der Faden 5. Durch Ziehen daran kann der Hohlkörpen 2 aus der Harnröhre 1 herausgeholt werden. Durch die Zugkraft wird der Hohlkörper 2 gestreckt und dadurch länger und schmäler. Einerseits kann er so leicht in das Halsteil 7 gleiten und durch dieses nach aussen gezogen werden. Gleichzeitig wird der Hohlkörper 2, durch das Längen, auf einer bestimmten Fläche von der Harnröhrenwand 9 gelöst und kann dadurch ohne grossen Widerstand schmerzfrei nach aussen bewegt werden.

## Patentansprüche

1. Schutzvorrichtung für den unkontrollierten Abfluß von Urin bei Inkontinenz mit einen, in einer Harnröhre anordenbaren länglichen Verschluß, der von einem Hohlkörper gebildet ist, der an einem Ende offen ausgebildet ist
**dadurch gekennzeichnet**,
daß der Hohlkörper(2)an seinem distalen Ende (3) geschlossen und an seinem proximalen Ende (4) offen ausgebildet ist.

2. Schutzvorrichtung nach Anspruch 1
**dadurch gekennzeichnet**,
daß der Hohlkörper (2) nicht länger als die Harnröhre (1) ausgebildet ist, wobei der Hohlkörper (2) so in der Harnröhre (1) angeordnet ist, daß das distale Ende (3) des Hohlkörpers (2) flächig am Halsteil (7) der Harnröhre (1) angeordnet ist.

3. Schutzvorrichtung nach Anspruch 1 oder 2
**dadurch gekennzeichnet**,
daß der Hohlkörper (2) annähernd die Form eines elliptischen Paraboloids aufweist.

4. Schutzvorrichtung nach einem der Ansprüche 1 bis 3
**dadurch gekennzeichnet**,
daß der Hohlkörper (2) annähernd zylindrisch oder kegelförmig ausgebildet ist.

5. Schutzvorrichtung nach einem der Ansprüche 1 bis 4
**dadurch gekennzeichnet**,
daß der Hohlkörper (2) aus einem elastischen Material gebildet ist.

6. Schutzvorrichtung nach einem der Ansprüche 1 bis 5
**dadurch gekennzeichnet**,
daß der Hohlkörper (2) eine Wandstärke aufweist, die im Bereich des distalen Endes (3) dicker als im Bereich des proximalen Endes (4) ausgebildet ist.

7. Schutzvorrichtung nach einem der Ansprüche 1 bis 6
**dadurch gekennzeichnet**,
daß am distalen Ende (3) des Hohlkörpers (2) eine Zugeinrichtung, insbesondere ein Faden (5) angeordnet ist.
